# FASCICULE DE BREVET EUROPEEN

(11) **EP 4 010 071 B1**
(45) Date de publication et mention de la délivrance du brevet: **16.04.2025**
(21) Numéro de dépôt: 20747434.7
(22) Date de dépôt: 04.08.2020
(51) Int. Cl.: A61N 2/02

(54) **DISPOSITIF POUR L'ÉMISSION D'UN CHAMP MAGNÉTIQUE**
VORRICHTUNG ZUM AUSSENDEN EINES MAGNETFELDES
DEVICE FOR EMITTING A MAGNETIC FIELD

(30) Priorité: 05.08.2019 FR 1908980
(43) Date de publication de la demande: 15.06.2022
(73) Titulaire: Cosmosoft, 75017 Paris (FR)
(72) Inventeur: GREFF, Daniel, 78490 Mere (FR)
(74) Mandataire: Cabinet Beau de Loménie
(86) Numéro de dépôt international: PCT/EP2020/071926
(87) Numéro de publication internationale: WO 2021/023749

(56) Documents cités:
- EP-A1- 2 665 516
- DE-U1- 202010 012 057
- KR-A- 20080 061 463
- US-A1- 2010 130 945
- US-B1- 6 561 968
- MODI ANKITA ET AL: "Hexagonal coil systems for uniform magnetic field generation", 2016 IEEE ASIA-PACIFIC CONFERENCE ON APPLIED ELECTROMAGNETICS (APACE), IEEE, 11 December 2016 (2016-12-11), pages 47 - 51, XP033092090, DOI: 10.1109/APACE.2016.7916472

## Description

### Domaine Technique

La présente invention se rapporte au domaine général des dispositifs utilisant des ondes électromagnétiques pour agir sur le métabolisme du corps humain en vue d'induire des changements physiologiques, en particulier pour favoriser le sommeil, simuler une pratique sportive ou traiter les surcharges adipeuses.

### Technique antérieure

La réduction de la masse graisseuse des tissus cutanés superficiels par application d'un champ magnétique a déjà été proposée. On pourra notamment se référer aux documents FR 2,855,415, FR 2,906,727, EP 2,068,810, qui décrivent des dispositifs composés de sangles isolantes appliquées sur la partie du corps humain à traiter, ces sangles étant équipées d'un conducteur replié en brins successifs et relié à un générateur de tension alternatif pour créer des ondes électromagnétiques.

Des dispositifs permettant de stimuler la lipolyse de la masse graisseuse intramusculaire et viscérale par émission de champs électromagnétiques de basse fréquence ont été proposés plus récemment dans la demande EP 2,665,516. Ces dispositifs comprennent des antennes dipolaires de forme rectangulaire.

La demande US 2010/130945 a proposé un procédé de traitement du myocarde en cas de détresse ischémique en exposant le myocarde à un dispositif de bobines émettant un champ magnétique alternatif de fréquence multiple de 16 Hz ou multiple de 8 Hz pour activer les canaux ATP potassiques dans le myocarde. Enfin, des dispositifs comprenant des bobines hexagonales ont été décrits dans les demandes DE 2010 012 057 et KR 2008 061 463, et Modi Ankita et al.ont proposé un modèle mathématique de modélisation du champ magnétique créé par de telles bobines au cours de la conférence Asia-Pacific Conference on Applied Electromagnetics (APACE) du 11-13 décembre 2016.

Le besoin subsiste néanmoins d'améliorer les dispositifs existants.

### Objet et résumé de l'invention

Ce but est atteint grâce à un dispositif pour l'émission d'un champ magnétique selon la revendication 1. Le dispositif comprend:
- un support isolant destiné à être mis en contact avec une partie du corps d'une personne,
- au moins une première antenne et au moins une deuxième antenne, chaque antenne étant une bobine d'un fil métallique conducteur enroulé suivant le tracé d'un hexagone, la longueur de son côté étant comprise entre 2 cm et 20 cm, et
- une source d'alimentation en courant de l'antenne,
la première antenne, la deuxième antenne et la source d'alimentation étant solidaires du support isolant, le courant étant alternatif et la première antenne et la deuxième antenne étant bobinées en sens inverse.

Le courant étant alternatif, la première antenne émet donc un champ magnétique de sens opposé à celui émis par la deuxième antenne, et le sens du champ magnétique émis par chaque antenne alterne selon la fréquence du courant d'alimentation. Ce dispositif présente plusieurs avantages. Le fait de combiner deux bobines dont le sens d'enroulement du fil est inversé, et d'alimenter les deux bobines avec un courant alternatif permet d'émettre un champ magnétique global qui traverse le corps d'une personne sur lequel le dispositif est placé, pendant chaque demi-période du courant alternatif, et non pendant une période entière.

### Brève description des dessins

D'autres caractéristiques et avantages de la présente invention ressortiront de la description faite ci-dessous, en référence aux figures annexées.
La figure 1 est une vue d'une personne munie de trois dispositifs selon l'invention : un dispositif autour de chaque bras 200, un dispositif autour de la taille 100 et un dispositif autour de chaque jambe 300 comprenant plusieurs ensembles d'antennes;
La figure 2 est une vue détaillée d'un dispositif de l'invention qui peut être appliqué autour de la ceinture abdominale d'une personne ;
La figure 3 présente une vue d'un dispositif selon l'invention comprenant deux antennes bobinées en sens inverse, ainsi qu'un schéma représentant le profil des champs magnétiques émis par chacune des antennes;
La figure 4 est une vue de la partie du dispositif 300 de la figure 1 appliqué autour de la jambe gauche ;
La figure 5 est un dispositif destiné à être appliqué sur le contour du visage,
La figure 6 est un dispositif sous la forme d'un tapis;
La figure 7 est un schéma d'une antenne sous la forme d'un hexagone régulier de côté A ;
La figure 8 est un dispositif qui peut être appliqué autour des mains.

### Description détaillée des modes de réalisation

Le dispositif de l'invention comprend au moins deux antennes hexagonales.

On entend par « antenne hexagonale », une antenne plate dipolaire de forme hexagonale qui émet un champ magnétique suivant un vecteur perpendiculaire à sa surface. L'antenne de forme hexagonale est une bobine de fil métallique conducteur enroulé suivant le tracé d'un hexagone. Les angles de l'hexagone sont de préférence légèrement arrondis pour ne pas risquer de casser le fil conducteur. La ligne de métal conducteur est avantageusement dessinée le long du périmètre de l'hexagone et s'enroule sur plusieurs tours non jointifs, soit vers l'intérieur, soit vers l'extérieur, si bien que les spires ne sont pas en contact les unes avec les autres. Pour limiter la densité de courant dans le dispositif, le conducteur électrique peut comprendre au maximum une dizaine de tours afin d'assurer à la fois i) la sécurité de l'usager (limiter une élévation de température trop grande pouvant conduire à des brûlures) et ii) une intensité du champ magnétique suffisante. Chaque antenne permet de générer un champ dont la forme est assimilable à un cornet dont la section est hexagonale. Les antennes sont de préférence des bobines en fil de cuivre. L'épaisseur du fil métallique est de préférence comprise entre 200 et 300 microns.

Par « hexagonale », on entend une forme géométrique comprenant six côtés. Les longueurs des côtés de l'hexagone sont de préférence égales à plus ou moins 10% près. L'hexagone est de préférence un hexagone régulier, les six côtés ayant la même longueur. Un hexagone particulier comprend un plus petit axe reliant deux côtés opposés et un plus grand axe reliant deux sommets opposés, le plus petit axe et le plus grand axe étant perpendiculaires.

Le dispositif de l'invention comprend de préférence au moins un ensemble d'antennes, ledit ensemble d'antennes comprenant au moins une antenne hexagonale, à la condition que le dispositif comprenne au moins la première antenne et la deuxième antenne.

Le dispositif de l'invention peut comprendre au moins deux ensembles d'antennes, les ensembles étant reliés électriquement en parallèle ou en série. On préfère que les deux ensembles soient branchés en parallèle.

Selon une première variante, un ensemble d'antennes comprend de préférence au moins la première antenne et la deuxième antenne, la première antenne et la deuxième antenne étant bobinées en sens inverse. La première antenne et la deuxième antenne peuvent être directement connectées électriquement, ou non. Lorsque la première antenne et la deuxième antenne font partie d'un même ensemble, elles sont de préférence directement connectées électriquement.

Lorsque l'ensemble d'antennes comprend plus de deux antennes, on préfère que deux antennes qui sont directement connectées électriquement aient un sens de bobinage inverse. Dans un ensemble d'antennes comprenant plus de deux antennes reliées en série, on préfère donc connecter les antennes en alternant le sens de leur bobinage.

Selon une deuxième variante, la première antenne et la deuxième antenne (qui ont un sens de bobinage inverse) appartiennent à deux ensembles d'antennes distincts.

Dans un ensemble d'antennes, les antennes peuvent être disposées en sous-groupes d'au moins deux antennes, la distance entre les antennes contigües d'un sous-groupe étant inférieure à la distance entre deux antennes de deux sous-groupes différents.

Le support isolant peut être formé d'au moins deux feuilles d'un matériau isolant souple et fin, par exemple de deux feuilles d'un matériau polymère d'épaisseur de l'ordre de 200-300 microns.

Les antennes sont avantageusement placées à l'intérieur du support isolant pour ne pas entrer en contact avec le corps. Elles peuvent être par exemple placées entre les deux feuilles de matériau isolant décrites précédemment.

L'épaisseur du support isolant est choisie de telle sorte que la dissipation d'énergie qui se produit au cours du fonctionnement des antennes ne provoque pas une élévation trop importante de la température, et ne gêne pas la personne. Elle est par exemple de l'ordre de 400-600 microns.

Le support isolant peut avoir différentes formes, selon la partie du corps auquel le dispositif est destiné. Par exemple, le support peut avoir la forme d'une ceinture destinée à être enroulée autour de l'abdomen. Le support isolant peut être alternativement découpé sous la forme d'une sangle pour épouser le contour du visage au niveau du cou. Selon d'autres variantes, le support isolant est en forme de tapis sur lequel la personne peut être allongée, ou sous forme d'un bracelet que l'on enroule autour des mains, des bras ou des chevilles.

Les antennes du dispositif selon l'invention émettent un champ magnétique dirigé localement vers certaines zones du corps à traiter, à savoir en particulier la ceinture abdominale, le visage, les bras, les mains, les cuisses, les mollets, les chevilles et le dos.

L'application de ce champ magnétique permet de simuler une activité sportive, notamment par son action sur les canaux calciques et ce, sans contraction musculaire ressentie par le patient. Ce dispositif permet non seulement de réduire la masse graisseuse sous-cutanée, viscérale et intermusculaire, mais aussi de lutter contre l'obésité, de lutter contre le syndrome métabolique, et d'augmenter de façon très significative les chances de procréation médicalement assistée chez des personnes stériles.

Le dispositif, lorsqu'il est placé sur le corps d'une personne saine, aura un effet non thérapeutique, bien qu'agissant sur le métabolisme. Il pourra avoir un effet esthétique en réduisant ma graisseuse sous-cutanée, ou simuler une activité sportive.

Chez une personne atteinte d'une maladie, notamment une personne atteinte d'obésité, le dispositif aura un effet thérapeutique sur la maladie à traiter.

Lorsque le dispositif est une ceinture abdominale, la composante principale du champ magnétique émis par chaque antenne n'est pas coaxiale à la colonne vertébrale de la personne. Par ailleurs, l'intensité du champ magnétique auquel sont exposées des zones critiques du corps (cœur, poumon, cerveau) est très faible, de sorte que l'utilisation du dispositif selon l'invention ne présente que peu de risques pour la santé de la personne.

La source d'alimentation émet de préférence un courant d'alimentation alternatif dont la fréquence peut être comprise entre 10 Hz et 100 Hz, de préférence comprise entre 40 Hz et 60 Hz. Elle peut être égale à 16 Hz, 32 Hz, 48 Hz, 64 Hz, 80 Hz ou 96 Hz, de préférence de l'ordre de 50 Hz. Par courant alternatif, on entend que la valeur et le sens du courant varient en fonction du temps, de préférence selon une fonction sinusoïdale.

Lorsque le courant d'alimentation est un courant alternatif, celui-ci présente de préférence une tension de l'ordre de 5 V à 25 V, par exemple de 10 V ou 20 V environ, et une intensité comprise entre 0,3 A et 1,5 A, par exemple de l'ordre de 0,5 A. En effet, il a été constaté qu'un courant d'alimentation présentant de telles caractéristiques permet d'obtenir les meilleurs résultats en termes de densité de champ magnétique émis ainsi que de densité d'énergie évitant de placer le patient dans une situation de risques électriques ou thermiques.

De façon avantageuse, les antennes sont dimensionnées pour émettre un champ magnétique dont l'intensité est au maximum de 600 microTesla (600 microT, soit 6 Gauss). Il a en effet été constaté que l'application d'un champ magnétique d'intensité supérieure à 600 microT peut présenter, à hautes doses, des effets secondaires nuisibles sur les cellules du cerveau et des os de la personne. La limitation à un tel seuil d'intensité permet donc d'éviter de tels risques.

Le dispositif de l'invention peut comprendre au moins trois antennes disposées selon un pavage.

De façon indépendante, la première antenne et la deuxième antenne peuvent être alignées selon leur plus grand axe ou selon leur plus petit axe.

Un dispositif de l'invention peut comprendre au moins deux ensembles d'antennes reliés en parallèle, chaque ensemble comprenant au moins deux antennes connectées en série et comprenant au moins la première antenne et la deuxième antenne. Dans ce cas, le dispositif peut être sous la forme d'une ceinture ou d'un pantalon.

Un dispositif de l'invention peut comprendre au moins deux ensembles d'antennes tels que les centres des antennes d'un même ensemble sont positionnées sur un même axe, que les axes des ensembles sont parallèles, et que chaque ensemble comprend au moins la première antenne et la deuxième antenne. Dans ce cas, le dispositif peut être sous la forme d'une ceinture ou d'un pantalon.

Par exemple, le dispositif est une ceinture abdominale comprenant un support de forme rectangulaire et deux ensembles d'antennes, les antennes d'un même ensemble étant alimentées en série, et les deux ensembles étant reliés en parallèle. Dans ce cas, le courant de la source d'alimentation est de préférence un courant alternatif, de sorte que les antennes du même sens de bobinage émettent un champ magnétique de sens opposé à celui émis par les antennes bobinées en sens opposé. Selon un mode de réalisation, les antennes d'un même ensemble sont alignées et les deux rangées d'antennes sont placées en quinconce.

Le dispositif peut comprendre un ensemble d'antennes reliées en série comprenant au moins la première antenne et la deuxième antenne, les antennes de l'ensemble d'antennes étant réparties par groupe de trois, et les antennes d'un groupe étant disposées en pavage.

Selon un autre mode de réalisation, le dispositif est une sangle visage destinée à être portée autour de l'ovale du visage, sur le cou. La sangle visage peut comprendre un support de forme courbe et un ensemble d'antennes reliées en série réparties en trois à cinq groupes, chaque groupe comprenant de une à cinq antennes, de préférence trois antennes disposées en pavage, deux d'entre elles étant bobinées en sens inverse. Dans cet ensemble d'antennes, une antenne bobinée dans le sens horaire peut être directement connectée à une antenne bobinée dans le sens anti-horaire.

Selon un autre mode de réalisation, le dispositif est un bracelet destiné à être portée autour du bras ou de la cheville, ledit bracelet comprenant un support de forme rectangulaire, la première antenne et la deuxième antenne. Le dispositif peut comprendre une troisième antenne dont le centre est aligné avec celui des deux autres.

Selon un autre mode de réalisation, le dispositif est une sangle destinée à être portée autour des mains, ladite sangle main comprenant un support de forme rectangulaire et un ensemble d'antennes comprenant trois premières antennes et trois deuxième antennes disposées selon le périmètre du support.

Selon encore un autre mode de réalisation, le dispositif est destiné à être placé autour de la jambe. Par exemple, il comprend au moins deux ensembles d'antennes, chaque ensemble comprenant au moins deux antennes, de préférence deux antennes bobinées en sens inverse, les centres des antennes d'un même ensemble étant positionnés sur un même axe, et les axes des ensembles étant parallèles. Un dispositif particulier comprend trois à cinq ensembles d'antennes destinés à la hanche, à la cuisse, au mollet et/ou à la cheville. Par exemple, le dispositif peut comprendre cinq ensembles dont un ensemble de trois antennes au niveau de la cheville, un ensemble de quatre antennes au niveau du mollet, deux ensembles au niveau de la cuisse composés respectivement de cinq antennes et de trois antennes, et un ensemble au niveau de la hanche composé de deux antennes.

Le dispositif peut comprendre un ensemble d'antennes comprenant au moins cinq premières antennes et aux moins cinq deuxièmes antennes, toutes alimentées en série et disposées en pavage.

Selon un dernier mode de réalisation, le dispositif est sous la forme d'un tapis sur lequel la personne peut s'allonger. Le tapis peut être posé sur le matelas d'un lit ou sur le sol. Il peut servir de tapis de relaxation ou de gymnastique. Le tapis sera de préférence placé de telle sorte que les antennes soient situées à proximité immédiate du dos, de préférence encore du bas du dos vers le milieu du dos. Dans un mode de réalisation particulier, le tapis comprend entre 10 et 25 antennes, ou entre 15 et 25 antennes. Le dispositif est par exemple sous la forme d'un tapis rectangulaire doté d'un seul ensemble d'antennes comprenant au moins dix antennes disposées en pavage et alimentées en série. Les antennes placées en pavage peuvent définir plusieurs rangées de deux ou trois antennes, les rangées étant disposées en quinconce.

Le dispositif de l'invention peut comprendre en outre un boîtier de contrôle de la source d'alimentation en courant alternatif des antennes et au moins un capteur de mesure du champ magnétique disposé sur le support et relié audit boîtier de contrôle. La présence d'un ou plusieurs capteurs de mesure du champ magnétique permet de déterminer avec précision le seuil d'induction magnétique appliquée à la personne. En fonction de ces mesures, un réglage peut donc être réalisé à l'aide du boîtier de contrôle.

Les dispositifs de l'invention ont pour but d'émettre des champs magnétiques dont les caractéristiques (notamment de direction et d'intensité) visent à réduire la masse graisseuse présente dans les parties du corps visées. Cette réduction des surcharges adipeuses est rendue possible grâce à l'effet du champ magnétique généré par le dispositif selon l'invention qui simule une pratique sportive.

Quelle que soit la partie du corps sur laquelle il est appliqué, le dispositif selon l'invention comprend i) un support isolant, ii) au moins deux antennes plates dipolaires de forme hexagonale qui sont bobinées en sens inverse et qui émettent un champ magnétique suivant un vecteur perpendiculaire à sa surface, et iii) une source d'alimentation en courant des antennes, le courant étant de préférence alternatif.

Chaque antenne est avantageusement dimensionnée pour exposer le corps de la personne à un champ magnétique dont l'intensité est au maximum de 600 microT environ. Une fois le dispositif appliqué au contact de la personne, le champ magnétique doit être de préférence de 600 microT au maximum en surface de peau et ne pas dépasser 1 microT au centre du corps. L'application d'un champ magnétique d'intensité supérieure à 600 microT peut présenter, à hautes doses, des effets secondaires nuisibles sur les cellules du cerveau et des os de la personne. La limitation à un tel seuil d'intensité de 600 microT permet donc d'éviter de tels risques.

Les côtés des antennes hexagonales ont de préférence une longueur comprise entre 3 cm et 15 cm, et de préférence encore entre 4 cm et 7 cm. Un côté de longueur de l'ordre de 5 cm à 6 cm permet notamment une ouverture conditionnant la décroissance rapide de la puissance du champ magnétique en fonction de la profondeur dans le corps.

Les antennes d'un même ensemble peuvent être disposées de différentes façons, par exemple être alignées ou disposées selon un pavage. Elles peuvent être alignées selon leur plus petit axe (dans ce cas deux côtés de deux hexagones contigus se font face) ou être alignées selon leur plus grand axe (dans ce cas les sommets de deux hexagones contigus se font face).

Les antennes d'un même ensemble ont de préférence la même forme et les mêmes dimensions. Toutes les antennes du dispositif ont de préférence la même forme et les mêmes dimensions.

Les antennes sont de préférence disposées de telle sorte que la plus petite distance entre un côté d'une première antenne et un côté d'une deuxième antenne qui lui est contiguë est comprise entre 0,5 cm et 1,5 cm. Ainsi, la distance entre deux antennes contigües - qu'elles appartiennent ou non à un même ensemble - est de préférence comprise entre 0,5 cm et 2 cm, de préférence encore de l'ordre de 1 cm. Cette distance permet de résoudre les problématiques de sécurité intrinsèque au dispositif, aussi bien en terme électrique si le dispositif était abimé, qu'en termes de champ magnétique et de conformité des puissances magnétiques dans des sections du corps limitées, sur des profondeurs suffisantes mais limitées.

Dans un mode de réalisation, les antennes sont reliées électriquement entre elles de sorte que les antennes d'un même ensemble soient alimentées en série. L'ensemble d'antennes comprend de préférence au moins deux antennes connectées entre elles en série et alimentées en courant alternatif.

Lorsque le dispositif comprend deux ensembles d'au moins deux antennes, on préfère que l'alimentation soit réalisée de sorte que les antennes bobinées dans le même sens appartenant au premier ensemble émettent un champ magnétique suivant un vecteur opposé en direction au vecteur du champ magnétique émis par les antennes bobinées dans un sens opposé et appartenant au deuxième ensemble. Les lignes de champ magnétique provenant des antennes d'un même ensemble présentent ainsi une intensité maximale en surface de la peau pour décroître en profondeur dans le corps. Dans le cas où le dispositif est placé sur la ceinture abdominale, le champ magnétique généré par un tel dispositif permet un traitement optimum des zones musculaires situées à proximité des hanches et au niveau des abdominaux (l'intensité du champ étant concentrée dans ces zones). Plus en profondeur, l'intensité du champ magnétique étant réduite, les organes de la personne, et en particulier la colonne vertébrale, sont épargnés par l'émission du champ magnétique. De plus, les antennes du dispositif émettent un champ magnétique selon un vecteur perpendiculaire à leur surface, le champ magnétique rayonné en dessous et au-dessus étant extrêmement faible. Les organes critiques de la personne tels que le cœur et les poumons sont donc également épargnés, lorsque le dispositif est placé à leur proximité.

La source d'alimentation délivre de préférence un courant alternatif ou pulsé dont la fréquence est comprise entre 10 Hz et 100 Hz, de préférence comprise entre 40 et 60 Hz. Elle peut être égale à 16 Hz, 32 Hz, 48 Hz, 64 Hz, 80 Hz ou 96 Hz, de préférence de l'ordre de 50 Hz. De plus, lorsqu'il est alternatif, le courant d'alimentation présente avantageusement une tension comprise entre 5 V et 25 V, de préférence entre 5 V et 15 V. Elle peut aussi être égale à 20 V dans certains modes de réalisation. L'intensité du courant d'alimentation du dispositif est de préférence comprise entre 0,3 A et 1,5 A, de préférence encore entre 0,5 A et 1,5 A, plus préférentiellement égale à 0,5 A.

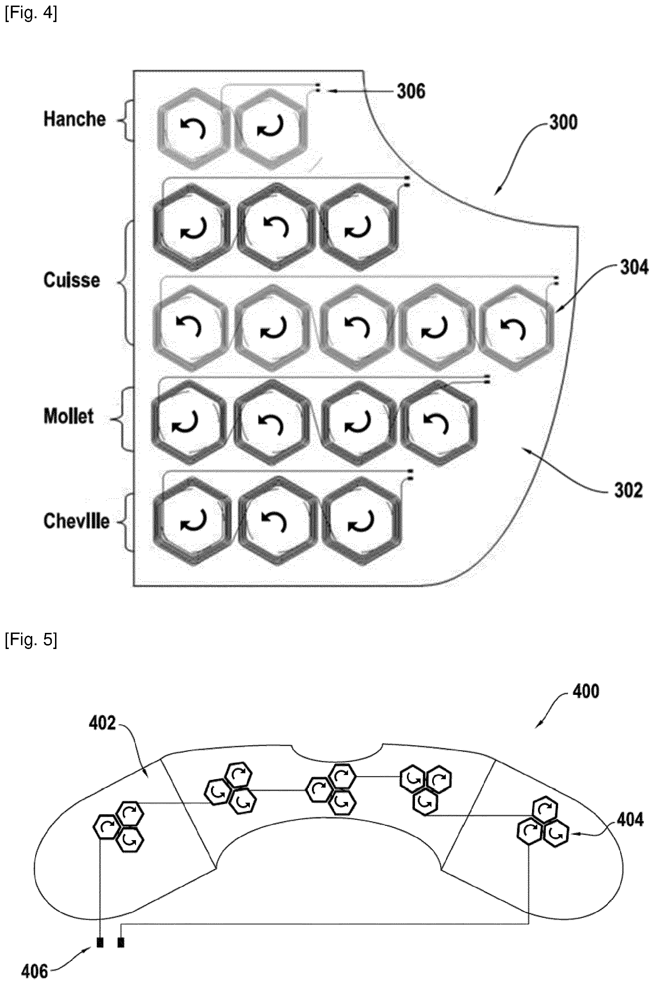

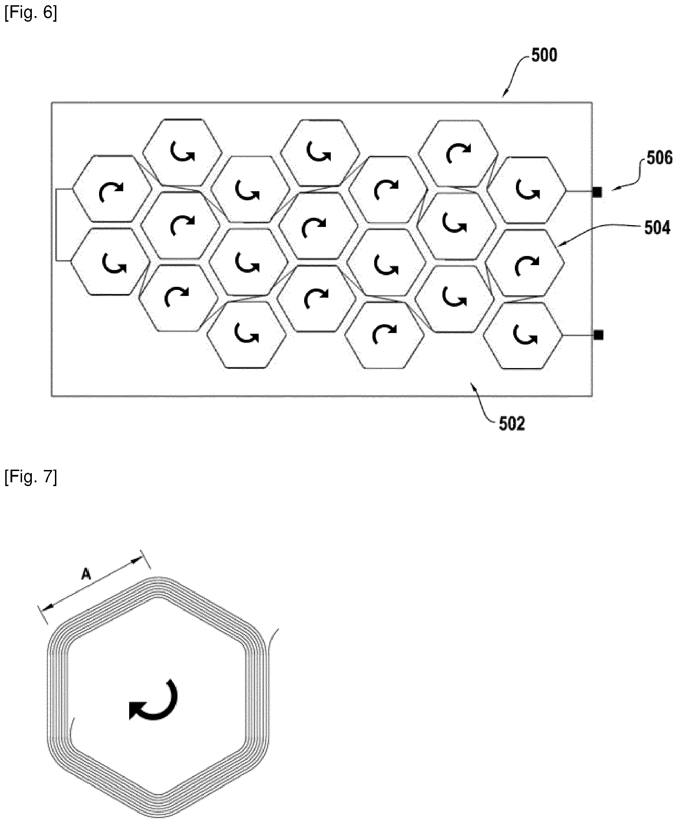

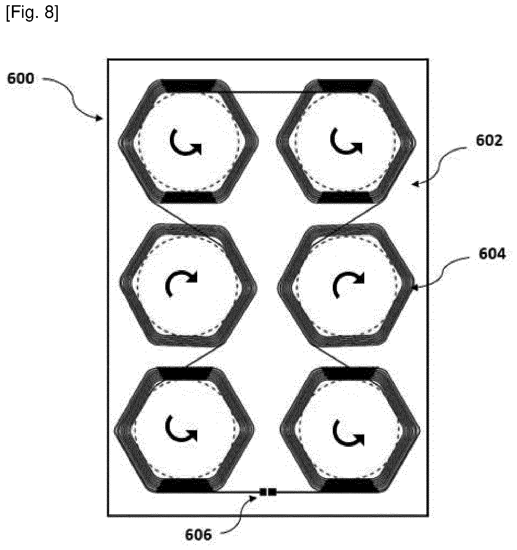

Le dispositif selon l'invention peut être appliqué au contact de différentes parties du corps d'une personne. Les figures illustrent ainsi plusieurs dispositifs, à savoir : un dispositif 100 disposé autour de la ceinture abdominale, des dispositifs 200 disposés autour des bras, et des dispositifs 300 disposés autour de chaque jambe de la personne. Le dispositif 400 de la Figure 5 est conçu pour être appliqué sur le cou, pour remodeler le contour du visage. Le dispositif de l'invention peut également être placé au contact du dos d'une personne, de préférence entre le bas et le milieu du dos, notamment en utilisant le dispositif 500 sous forme d'un tapis de la Figure 6. Le dispositif 600 de la Figure 8 est conçu pour être appliqué autour des mains, et son utilisation a notamment pour effet de rajeunir leur aspect, notamment en lissant la peau.

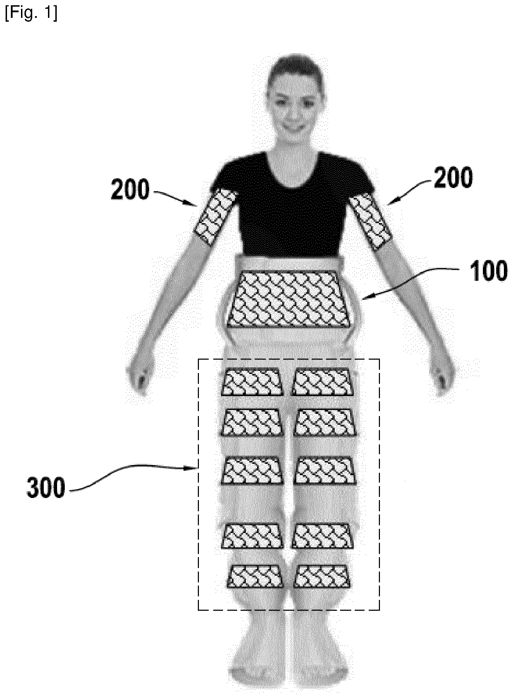

La Figure 1 représente une personne sur laquelle trois dispositifs ont été mis en place : un dispositif 100 en forme de ceinture disposé autour de la ceinture abdominale, deux dispositifs 200 en forme de bracelets disposés autour des bras, et un dispositif 300 en forme de pantalon enveloppant les jambes.

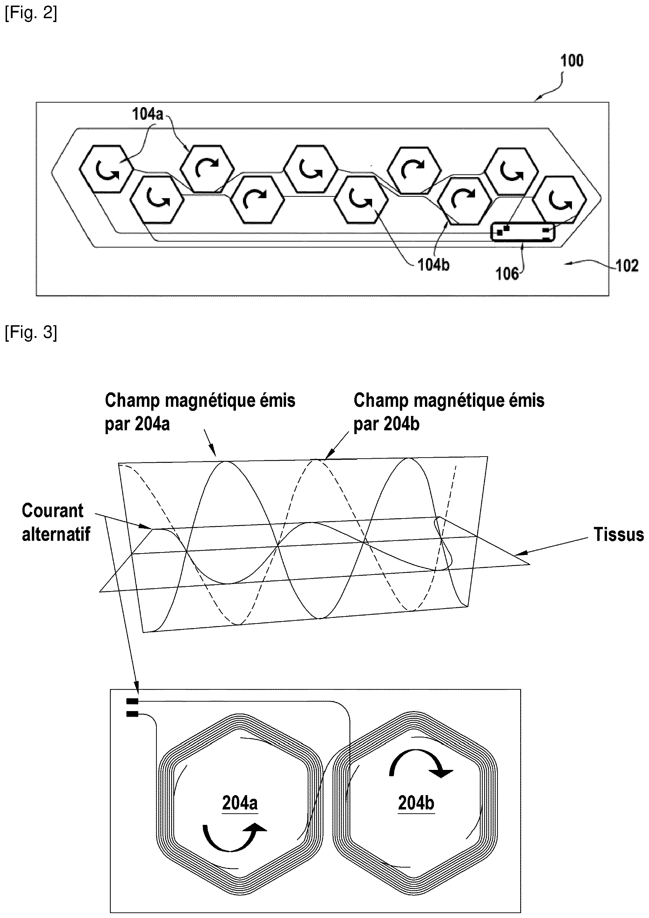

Comme représenté sur la Figure 2, le dispositif 100 destiné à être appliqué autour de la ceinture abdominale comprend un support 102 sous la forme d'une ceinture réalisé dans un matériau isolant, par exemple en PVC, et pouvant se fermer à la manière d'une ceinture vestimentaire. Le dispositif 100 comprend deux ensembles de cinq antennes, les antennes d'un même ensemble étant connectées en série et les deux ensembles étant connectés en parallèle. Chaque ensemble alterne une bobine enroulée dans le sens antihoraire (antenne 104a) et une bobine enroulée en sens horaire (antenne 104b). Les centres des antennes d'un même ensemble sont alignés suivant le plus grand axe des hexagones, et sont espacées d'un pas choisi de telle manière que les deux ensembles puissent être placés en cinquonce. La plus petite distance entre une antenne du premier ensemble et une antenne du deuxième ensemble va de préférence de 0,5 cm à 1,5 cm. La ceinture peut avoir une taille plus ou moins grande, par exemple celle d'une couverture ou d'un duvet dans lequel le patient peut être placé. Chaque antenne 104a, 104b est une antenne hexagonale plate ayant un pôle électromagnétique à chacun de ses sommets. Les antennes 104a et 104b du dispositif 100 de la ceinture abdominale sont alimentées en courant alternatif au moyen d'une source d'alimentation 106.

En liaison avec la Figure 3, on décrira maintenant le dispositif 200, qui pourra être disposé autour du bras de la personne. Ce dispositif 200 comprend un support 202 en matériau isolant, un ensemble d'antenne 204 comprenant deux antennes plates dipolaires de forme hexagonale, et une source d'alimentation 206 de l'ensemble d'antennes en courant alternatif. La première antenne 204a est une bobine dont le sens d'enroulement est inversé par rapport à celui de la bobine constituant la deuxième antenne 204b. Les deux antennes 204a et 204b sont placées côte à côte suivant leur plus petit axe à une distance de quelques centimètres l'une de l'autre. La disposition et la taille des antennes permettent d'émettre un premier champ magnétique dont les lignes de champ « traversent » le bras en étant annulées par celles du champ magnétique émis par la deuxième antenne. De la sorte, l'intensité du champ magnétique émis par les antennes peut être régulée en fonction du diamètre du bras de la personne. Si le patient a un bras de petit diamètre, l'influence du champ magnétique provenant du côté de l'antenne qui est opposé à celui enroulé est élevé et en direction opposée à celui-ci. Il permet ainsi d'annuler en partie le champ émis vers les tissus de la personne. Au contraire, si le patient a un bras de gros diamètre, l'antenne du dispositif est disposée de façon quasiment verticale par rapport à ce diamètre. L'antenne s'approche alors d'un simple élément radiant vertical dont la distance entre les extrémités devient peu significative grâce à la forme hélicoïdale de son enroulement.

En liaison avec la Figure 4, on décrira maintenant la moitié gauche du dispositif 300 destiné à être disposé autour des jambes d'une personne. Le dispositif 300 comprend cinq ensembles d'antennes sur la jambe gauche et cinq ensembles d'antennes sur la jambe droite, soit dix ensembles au total. Sur la Figure 4, cinq ensembles d'antennes 304 sont enveloppés dans un support isolant 302, et alimentés par une source de courant 306. Chaque ensemble alterne une antenne bobinée dans un sens et une antenne bobinée en sens inverse. Le support isolant du dispositif 300 est d'un seul tenant sous la forme d'un pantalon si bien que tous les ensembles d'antennes sont fixes les uns par rapport aux autres et peuvent être placés sur le patient en un seul geste. Alternativement, il est envisageable de réaliser un dispositif distinct pour chacune des parties de la jambe, à savoir un dispositif destiné à être appliqué sur la hanche, un sur le haut de la cuisse, un sur le bas de la cuisse, un sur le mollet, et un sur la cheville. Un dispositif au sens de l'invention est donc de préférence d'un seul tenant.

La Figure 5 représente une sangle visage destinée à être portée autour de l'ovale du visage, sur le cou. Le dispositif comprend un ensemble d'antennes plates dipolaires hexagonales. L'ensemble comprend cinq groupes de trois antennes, les antennes d'un même groupe étant disposées en pavage, et chaque groupe comprenant deux antennes bobinées en sens inverse.

Le dispositif peut être également un tapis conforme à celui dessiné sur la Figure 6 à l'échelle 1:6. Le tapis comprend 20 antennes de dimensions égales placées en série selon un pavage définissant des rangées de deux ou trois antennes disposées en quinconce.

Une antenne hexagonale faisant partie du dispositif de l'invention, et dont le fil conducteur est enroulé dans le sens horaire peut correspondre à celle représentée à la Figure 7. Le côté de l'hexagone régulier A peut être égal à 5 cm.

La Figure 8 représente un dispositif 600, qui est destiné à être porté autour de la main. Le dispositif comprend un ensemble d'antennes 604 comprenant six antennes plates dipolaires hexagonales enveloppées dans un support isolant 602 et alimentées en série en courant alternatif, deux antennes directement connectées étant bobinées en sens inverse et émettant un champ magnétique de sens opposé.

Les dispositifs de l'invention comprennent de préférence un boîtier de contrôle de la source d'alimentation en courant alternatif des antennes (non représenté). Ce boîtier de contrôle permet ainsi de contrôler l'intensité du champ magnétique émis pour les antennes du dispositif.

Par ailleurs, toujours de façon avantageuse, ces dispositifs peuvent comprendre au moins un capteur de mesure du champ magnétique (non représenté) disposé sur le support et relié au boîtier de contrôle. La présence d'un ou plusieurs capteurs de mesure du champ magnétique permet de déterminer avec précision le seuil d'induction magnétique appliquée au patient, qui correspond à l'intensité optimale du courant électrique à choisir. En fonction de ces mesures, un réglage peut donc être réalisé à l'aide du boîtier de contrôle.

En particulier, les dispositifs peuvent comprendre un moyen de réglage automatique de l'intensité du champ magnétique émis pour les antennes, en fonction de la morphologie des usagers. Ceci est réalisé par exemple en compensant la perméabilité magnétique des personnes que les dispositifs peuvent ajuster. Une dichotomie sur l'intensité du champ magnétique émis par les antennes permet de trouver le point de fonctionnement idéal. La dichotomie converge lorsque le champ magnétique mesuré du côté opposé de l'antenne est égal à par exemple 0,01 Gauss (1 microT).

Le dispositif de l'invention est utilisé pour l'émission d'un champ magnétique pénétrant dans les tissus d'au moins une partie d'au moins un membre et/ou du tronc d'une personne. Par « membre », on entend en particulier un ou les bras et une ou les jambes, et notamment le ou les mollets et le ou les cuisses, et en particulier la partie supérieure de la ou les cuisses. Par « tronc » on entend en particulier la zone abdominale, la zone lombaire et le cou.

Le dispositif de l'invention peut être appliqué sur au moins une partie du corps choisie parmi la tête, le visage, le cou, les bras, les mains, le dos, la taille, l'abdomen, la zone lombaire, les hanches, les cuisses, les mollets et les chevilles. Le dispositif peut être enroulé autour du corps, ou placé suivant un axe perpendiculaire, parallèle ou incliné par rapport à l'axe du membre ou de la partie du corps concerné.

Le faible niveau de champ magnétique requis en surface de la peau, entre un dixième et quelques fois le champ magnétique terrestre (0,5 Gauss équivalent à 50 microT), permet la mise en œuvre de ces techniques sans risques pour les cellules du corps humain, ni contraintes d'exploitation médicales.

Le dispositif, lorsqu'il est placé sur le corps d'une personne saine, n'aura aucun effet thérapeutique, bien qu'agissant sur le métabolisme. Il pourra avoir un effet esthétique en réduisant ma graisseuse sous-cutanée, avoir un effet relaxant, ou simuler une activité sportive.

Chez une personne atteinte d'une maladie, notamment une personne atteinte d'obésité, le dispositif aura un effet thérapeutique sur la maladie à traiter.

Le dispositif peut donc être utilisé dans des méthodes de traitement esthétique, des méthodes de traitement thérapeutique, des méthodes de relaxation ou des méthodes de simulation d'une activité sportive. Ces méthodes ne font pas partie de l'objet revendiqué et sont décrites uniquement à titre illustratif.

Le champ magnétique appliqué aux tissus vivants permet, notamment par son action sur les canaux calciques des cellules, de simuler une activité sportive sans que le patient ne ressente de contraction musculaire.

Ainsi est décrite une méthode pour simuler une pratique sportive chez une personne. La description concerne également une méthode pour réduire la masse graisseuse, notamment la masse graisseuse sous-cutanée, viscérale ou intramusculaire, et une méthode pour relaxer ou pour améliorer la qualité du sommeil. Chacune de ces trois méthodes comprend la mise en œuvre d'un champ magnétique sur au moins une partie d'au moins un membre d'une personne au moyen d'un dispositif tel que décrit précédemment.

Une méthode de relaxation musculaire ou d'amélioration de la qualité du sommeil, peut comprendre la mise en œuvre d'un champ magnétique sur au moins une partie d'au moins un membre et/ou du tronc d'une personne au moyen du dispositif décrit précédemment.

Dans chacune de ces méthodes, le dispositif peut prendre la forme d'une ceinture, d'une sangle, d'un bracelet, d'une couverture, d'une combinaison, d'un pantalon, d'une jambe de pantalon, ou d'un tapis.

Selon un mode de mise en œuvre particulier, la méthode permet de remodeler la silhouette ou d'améliorer l'esthétique du corps d'une personne saine, i.e. qui n'est atteinte d'aucune maladie déclarée en rapport avec une surcharge de masse adipeuse. Le dispositif peut être utilisé pour une méthode non-thérapeutique dans un but esthétique de réduction de la masse graisseuse viscérale ou intermusculaire qui comprend la mise en œuvre d'un champ magnétique sur au moins une partie d'au moins un membre et/ou du tronc d'une personne au moyen d'un dispositif décrit plus haut, en particulier d'une personne saine.

Le dispositif de l'invention permet une réduction de la masse graisseuse viscérale ou intra-musculaire, c'est-à-dire au niveau des adipocytes intra-musculaires. La réduction de la masse graisseuse en profondeur permet une amélioration très efficace de l'esthétique de la personne, par exemple de sa silhouette. Les dispositifs de l'art antérieur ne permettent pas de stimuler la lipolyse en profondeur dans les tissus. Au contraire, le champ magnétique généré par le dispositif de l'invention provoque la consommation de la graisse située en profondeur, notamment sous le derme, dans les viscères ou dans les muscles. Le champ magnétique généré par une antenne à la surface de la peau pénètre dans le corps jusqu'à une profondeur donnée, typiquement de l'ordre de 2 cm à 10 cm, de préférence à plus de 5 cm, sur toute la surface de l'antenne. Son intensité décroît avec la profondeur.

Grâce au dispositif de l'invention, il n'est pas nécessaire d'imposer en surface de la peau un champ magnétique d'intensité élevée pour assurer en profondeur une intensité suffisante pour activer les cibles biologiques. Les dispositifs de l'art antérieur ne permettent pas d'agir à une telle profondeur sans mettre en cause la sécurité de la personne. Il n'y a pas de diffusion du champ magnétique sur une zone du corps qui ne doit pas être exposée à un champ magnétique, telle que le cœur, le cerveau ou les poumons.

Le dispositif peut être utilisé pour le traitement ou la prévention de certaines maladies ou de troubles choisis parmi la stérilité, l'obésité et le syndrome métabolique, et permet notamment la prévention du diabète de type 2, des troubles cardiovasculaires, et des accidents vasculaires cérébraux.

Un dispositif tel que décrit précédemment peut être utilisé dans le traitement ou la prévention de la stérilité, de l'obésité ou du syndrome métabolique.

Le syndrome métabolique détecté chez une personne n'ayant déclaré aucun symptôme particulier correspond à un risque de désorganisation métabolique supérieur à un individu dit en bonne santé. On peut ainsi définir le syndrome métabolique comme un risque d'accident cardio-vasculaire multiplié par trois par rapport à un individu réellement en bonne santé. Le syndrome métabolique décrit un état qui est considéré comme préfigurant plusieurs maladies graves dont le diabète de type 2 (DT 2) ; les troubles cardiovasculaires ; et les accidents vasculaires cérébraux (AVC).

Le dispositif de l'invention permet également de prévenir ou de lutter contre l'obésité, en particulier l'obésité abdominale, et par voie de conséquence contre les maladies associées à l'obésité.

Un champ magnétique présentant l'intensité et la fréquence mentionnées plus haut possède également une activité pour stimuler et/ou améliorer l'ovogenèse et la spermatogenèse. Ainsi l'utilisation du dispositif de l'invention peut provoquer ou augmenter l'ovogenèse et/ou la spermatogenèse. Le dispositif permet donc de traiter la stérilité, d'augmenter la fertilité, et de prévenir un risque de stérilité, notamment lorsqu'elle découle d'une obésité ou d'un risque d'obésité élevé contre lequel il faut lutter. Le champ magnétique généré par le dispositif décrit plus haut peut améliorer la fertilité chez des femmes ou des hommes présentant un Indice de Masse Corporelle (IMC) supérieur ou égal à 30.

Par « traitement ou prévention de la stérilité », on entend la stimulation ou l'augmentation de l'ovogenèse ou de la spermatogenèse, notamment dans le cas où celle-ci est inexistante ou lorsqu'elle est insuffisante. Le traitement de la stérilité entraîne l'augmentation du taux de grossesse chez des personnes suivant un protocole de procréation médicalement assistée.

On pourra adapter les paramètres de dimensions de l'antenne, d'intensité et de tension du courant électrique alimentant l'antenne, de durée des séances d'exposition au champ magnétique et du nombre de séances en fonction du résultat recherché.

Selon un mode de réalisation avantageux, un contrôle du dosage d'un champ magnétique appliqué sur le patient peut être réalisé. Ce contrôle du dosage est en particulier effectué par régulation de l'intensité du champ magnétique. Ce contrôle du dosage est facilement effectué par une variation de l'intensité du courant électrique alimentant les antennes et générant le champ magnétique, qui est typiquement comprise entre 0,3 A et 1,5 A. Le dosage d'un champ magnétique appliqué sur une personne, notamment dans un but de soin esthétique ou thérapeutique, peut être réalisé par disposition d'un capteur d'ondes magnétiques à proximité immédiate d'une antenne rayonnante, ledit capteur d'ondes magnétiques étant en communication avec un boîtier de contrôle du champ magnétique, ledit boîtier de contrôle communiquant avec une ou plusieurs antennes rayonnantes, et la mesure par le boîtier de contrôle de la perméabilité magnétique de la personne afin d'adapter le champ magnétique émis par l'antenne rayonnantes en fonction d'un profil d'individu, notamment en diminuant ou augmentant l'intensité du courant. Ceci permet notamment un dosage en intensité et en durée du champ magnétique appliqué à la personne.

Une mesure de la réponse des tissus d'une personne à une mise en contact avec un champ magnétique, notamment dans un but d'optimisation d'un soin esthétique ou thérapeutique par champ magnétique, peut comprendre la variation des fréquences du champ électromagnétique, de préférence entre 10 Hz et 10 kHz, au regard d'une ou plusieurs parties d'un ou plusieurs membres et/ou d'une ou plusieurs parties du tronc d'une personne, et la mesure du champ électromagnétique pour estimer la réponse des tissus de la personne. La composante de champ électrique, présente dans tout champ électromagnétique, devient significative à partir de 1 kHz, alors qu'à 50 Hz le champ électromagnétique est surtout caractérisé par un champ magnétique prédominent. Ce champ électrique permet alors d'effectuer une mesure fine de la perméabilité magnétique.

Cette mesure permet notamment l'estimation de la masse graisseuse et/ou de la masse musculaire afin notamment d'adapter l'intensité et la durée du champ magnétique appliquée à la personne.

La mesure peut être par exemple mise en œuvre en faisant passer un courant alternatif dans l'ensemble des antennes, puis l'extinction successive ou simultanée d'une ou plusieurs antennes pour mesurer le champ électromagnétique émis par les antennes restantes. Ainsi, en mesurant le champ électromagnétique émis au travers des tissus d'une personne, par exemple en positionnant le capteur électromagnétique de manière diamétralement opposée à l'antenne émettrice du champ à mesurer, le champ électromagnétique capté modélise la perméabilité magnétique de la personne. Dans le cas du dispositif de la Figure 2, on peut facilement ne laisser émettre que les antennes dorsales et mesurer le champ qui est reçu dans la partie abdominale. Un dispositif sous la forme d'une ceinture peut donc comprendre un capteur électromagnétique disposé de manière diamétralement opposée à au moins une antenne lorsque que la ceinture est mise en position sur le patient. Le capteur électromagnétique peut être une des antennes opposées, mais utilisée en réception pour détecter le niveau de signal traversant le patient.

Il est alors possible de distinguer des rapports de permittivité diélectrique de plusieurs ordres de magnitude entre 10 Hz et 10 kHz, mesurables réellement entre 1 kHz et 10 kHz. On peut réaliser une courbe de variation de permittivité diélectrique de la graisse (mesurée sur la poitrine par exemple) en fonction de la fréquence et une autre courbe de variation de permittivité diélectrique du tissu musculaire en fonction de la fréquence. La présence d'une pente de courbe type graisse plutôt que muscle permet d'évaluer les ratios de tissus à traiter. Cette information est complétée par la connaissance des poids et taille de la personne.

On peut également réaliser l'estimation de la réponse des tissus de la personne en faisant varier les fréquences du champ magnétique sur une ou plusieurs parties d'un ou plusieurs membres et/ou parties du tronc de la personne. Par exemple la mesure est effectuée par l'intermédiaire d'un capteur magnétique, et par exemple un capteur à effet hall ou magnéto résistif, notamment pour déterminer le seuil de puissance maximum et/ou optimum du champ magnétique à appliquer à la personne.

Le dispositif de l'invention permet d'émettre un champ magnétique alternatif ciblé sur certaines zones du corps, et notamment situées dans les zones de forte densité de muscles et/ou viscères. L'action du champ magnétique consiste à interagir avec certains éléments intervenant dans les cycles de consommation des graisses stockées dans le corps. En particulier les cycles organiques liés au calcium sont suractivés.

L'invention est illustrée par l'Exemple qui suit.

### Exemple 1 : Dispositifs de l'invention et comparaison avec l'art antérieur

### 1. Dispositifs utilisés

Dans un premier temps, on a appliqué sur une première personne plusieurs dispositifs conformes à l'invention:
- une ceinture abdominale conforme à la description de la Figure 2,
- un bracelet autour de chaque bras conforme à la description de la Figure 3,
- une enveloppe autour de chaque jambe conforme à la Figure 4.
- sur le contour du visage de la personne, un dispositif conforme à la description de la Figure 5.

Une deuxième personne a été allongée sur un tapis conforme au dessin de la Figure 6.

Les antennes hexagonales de ces cinq dispositifs étaient toutes des hexagones réguliers de 5 cm de côté.

Dans un second temps, à titre de comparaison, on a appliqué sur la première personne plusieurs dispositifs de la demande EP 2,665,516 de l'art antérieur, soit :
- une ceinture abdominale comprenant deux ensembles parallèles de trois antennes plates dipolaires rectangulaires et disposées dans le sens de la longueur de la ceinture,
- une ceinture autour de chaque bras et autour de chaque cheville, ladite ceinture comprenant un ensemble de deux antennes plates dipolaires rectangulaires parallèles disposées dans le sens de la longueur de la ceinture,
- deux ceintures autour de chaque jambe dont une ceinture sur le haut des cuisses et une ceinture sur les mollets, chaque ceinture comprenant deux antennes plates dipolaires rectangulaires parallèles dans le sens de la longueur,
- une sangle autour de l'ovale du visage, comprenant deux antennes plates dipolaires rectangulaires.

Les antennes plates dipolaires rectangulaires étaient toutes de la même dimension (35 cm de longueur et 3 cm de largeur).

Le courant d'alimentation des dispositifs de l'invention et des dispositifs de l'art antérieur était un courant alternatif de 50 Hz, 0,5 A et 10 V.

### 2. Mesure de l'intensité du champ magnétique émis

Pour chacun des dispositifs décrits précédemment, on a mesuré l'intensité du champ magnétique émis en trois points différents avec un Gaussmètre de référence GM07 pré-étalonné, puis on a calculé la moyenne des trois mesures.

Les résultats sont présentés dans le Tableau 1 ci-dessous.

**[Tableau 1]**

| Champ magnétique (Gauss) | | | | | | | | |
|---|---|---|---|---|---|---|---|---|
| | Art antérieur | | | | Invention | | | |
| Dispositif | Point 1 | Point 2 | Point 3 | Moyenne | Point 1 | Point 2 | Point 3 | Moyen ne |
| **Dispositif Bras** | 0.6 | 0.5 | 0.7 | 0.6 | 3.5 | 3.39 | 3.21 | 3.36 |

| **Dispositif Ceinture Abdominale** | | | | | | | | |
|---|---|---|---|---|---|---|---|---|
| Ensemble n°1 Ceinture Abdominale | 1 | 1.1 | 1 | 1.03 | 4.07 | 3.90 | 3.65 | 3.87 |
| Ensemble n°2 Ceinture Abdominale | 0.7 | 0.7 | 0.8 | 0.73 | 3.2 | 3.8 | 3.75 | 3.58 |

| **Dispositif Jambe** | | | | | | | | |
|---|---|---|---|---|---|---|---|---|
| Ensemble n°1 Cuisse | 0.8 | 1 | 0.9 | 0.9 | 5.07 | 4.85 | 5.26 | 5.06 |
| Ensemble n°2 Cuisse | 0 | 0 | 0 | 0 | 4.4 | 4.64 | 5.43 | 4.82 |
| Ensemble Mollet | 0.9 | 0.6 | 0.7 | 0,73 | 3.15 | 4.20 | 4.50 | 3.95 |
| Ensemble Cheville | 0 | 0 | 0 | 0 | 2.96 | 3.25 | 4.11 | 3.44 |
| **Dispositif Visage** | 0.3 | 0.5 | 0.4 | 0.4 | 1.5 | 2.5 | 2 | 2.00 |
| **Dispositif Tapis** | 1 | 1.1 | 1 | 1.03 | 4.5 | 6.0 | 3 | 4.50 |

La valeur du champ magnétique du dispositif de l'invention est supérieure à celle du dispositif de l'art antérieur.

En outre, les dispositifs de l'invention permettent de couvrir 70% du corps, tandis que le dispositif de l'art antérieur ne permet de ne couvrir que 30% du corps.

## Revendications

1. Dispositif (100 ; 200 ; 300 ; 400 ; 500 ; 600) pour l'émission d'un champ magnétique comprenant :
- un support isolant (102 ; 202 ; 302 ; 402 ; 502 ; 602) destiné à être mis en contact avec une partie du corps d'une personne,
- au moins une première antenne (104a ; 204a) et au moins une deuxième antenne (104b ; 204 b), chaque antenne étant une bobine d'un fil métallique conducteur enroulé suivant le tracé d'un hexagone, la longueur de son côté étant comprise entre 2 cm et 20 cm,
- une source d'alimentation (106 ; 206 ; 306 ; 406 ; 506 ; 606) en courant de l'antenne,
la première antenne, la deuxième antenne et la source d'alimentation étant solidaires du support isolant,
le courant étant alternatif et
la première antenne et la deuxième antenne étant bobinées en sens inverse.

2. Dispositif selon la revendication 1, **caractérisé en ce que** la longueur du côté de l'hexagone est comprise entre 3 cm et 15 cm.

3. Dispositif selon la revendication 1, **caractérisé en ce qu'**il comprend au moins trois antennes disposées selon un pavage.

4. Dispositif selon la revendication 1, **caractérisé en ce que** la première antenne et la deuxième antenne sont alignées selon leur plus grand axe.

5. Dispositif selon la revendication 1, **caractérisé en ce que** la première antenne et la deuxième antenne sont alignées selon leur plus petit axe.

6. Dispositif selon la revendication 1, **caractérisé en ce que** la première antenne et la deuxième antenne sont directement connectées électriquement.

7. Dispositif selon la revendication 1, **caractérisé en ce que** la partie du corps est choisie dans le groupe constitué par la tête, le visage, le cou, les mains, les bras, le dos, la taille, l'abdomen, la zone lombaire, les hanches, les cuisses, les mollets et les chevilles.

8. Dispositif selon la revendication 1, sous la forme d'une ceinture ou d'un pantalon, **caractérisé en ce qu'**il comprend au moins deux ensembles d'antennes reliés en parallèle, **en ce que** chaque ensemble comprenant au moins deux antennes connectées en série et **en ce que** chaque ensemble comprend au moins la première antenne et la deuxième antenne.

9. Dispositif selon la revendication 1, sous la forme d'une sangle visage comprenant un ensemble d'antennes reliées en série comprenant au moins la première antenne et la deuxième antenne, les antennes de l'ensemble étant réparties par groupe de trois, et les antennes d'un groupe étant disposées en pavage.

10. Dispositif selon la revendication 1, **caractérisé en ce qu'**il est sous la forme d'un tapis rectangulaire sur lequel la personne peut être allongée, et qu'il comprend un ensemble d'antennes comprenant au moins cinq premières antennes et aux moins cinq deuxièmes antennes, toutes alimentées en série et disposées en pavage.

11. Dispositif selon la revendication 1, **caractérisé en ce qu'**il est sous la forme d'une sangle destinée à être portée autour des mains, ladite sangle main comprenant un support de forme rectangulaire et un ensemble d'antennes comprenant trois premières antennes et trois deuxièmes antennes disposées selon le périmètre du support, la première antenne alternant avec la deuxième antenne dans l'ensemble.

12. Dispositif selon la revendication 1, **caractérisé en ce qu'**il est sous la forme d'un bracelet destiné à être portée autour du bras ou de la cheville, ledit bracelet comprenant un support de forme rectangulaire, la première antenne et la deuxième antenne.

## Patentansprüche

1. Vorrichtung (100; 200; 300; 400; 500; 600) zum Senden eines Magnetfeldes, umfassend:
- einen isolierenden Träger (102; 202; 302; 402; 502; 602), der dazu bestimmt ist, mit einem Teil des Körpers einer Person in Kontakt gebracht zu sein,
- mindestens eine erste Antenne (104a; 204a) und mindestens eine zweite Antenne (104b; 204b), wobei jede Antenne eine Spule aus einem leitenden Metalldraht ist, der entsprechend dem Verlauf eines Sechsecks gewickelt ist, wobei die Länge seiner Seite zwischen 2 cm und 20 cm liegt,
- eine Stromversorgungsquelle (106; 206; 306; 406; 506; 606) für die Antenne,
wobei die erste Antenne, die zweite Antenne und die Stromversorgungsquelle fest mit dem isolierenden Träger verbunden sind,
wobei der Strom Wechselstrom ist und die erste Antenne und die zweite Antenne in entgegengesetzter Richtung gewickelt sind.

2. Vorrichtung nach Anspruch 1, **dadurch gekennzeichnet, dass** die Länge der Seite des Sechsecks zwischen 3 cm und 15 cm liegt.

3. Vorrichtung nach Anspruch 1, **dadurch gekennzeichnet, dass** sie mindestens drei Antennen umfasst, die gemäß einer Pflasterung angeordnet sind.

4. Vorrichtung nach Anspruch 1, **dadurch gekennzeichnet, dass** die erste Antenne und die zweite Antenne gemäß ihrer größten Achse ausgerichtet sind.

5. Vorrichtung nach Anspruch 1, **dadurch gekennzeichnet, dass** die erste Antenne und die zweite Antenne gemäß ihrer kleinsten Achse ausgerichtet sind.

6. Vorrichtung nach Anspruch 1, **dadurch gekennzeichnet, dass** die erste Antenne und die zweite Antenne direkt elektrisch verbunden sind.

7. Vorrichtung nach Anspruch 1, **dadurch gekennzeichnet, dass** der Körperteil aus der Gruppe ausgewählt ist, die aus Kopf, Gesicht, Hals, Händen, Armen, Rücken, Taille, Bauch, Lendenbereich, Hüften, Oberschenkeln, Waden und Knöcheln besteht.

8. Vorrichtung nach Anspruch 1 in Form eines Gürtels oder einer Hose, **dadurch gekennzeichnet, dass** sie mindestens zwei Antennenanordnungen umfasst, die parallel verbunden sind, dass jede Anordnung mindestens zwei in Reihe geschaltete Antennen umfasst und dass jede Anordnung mindestens die erste Antenne und die zweite Antenne umfasst.

9. Vorrichtung nach Anspruch 1 in Form eines Gesichtsriemens, umfassend eine Anordnung von in Reihe geschaltete Antennen, die mindestens die erste Antenne und die zweite Antenne umfasst, wobei die Antennen der Anordnung in Dreiergruppen verteilt sind und die Antennen einer Gruppe pflasterartig angeordnet sind.

10. Vorrichtung nach Anspruch 1, **dadurch gekennzeichnet, dass** sie in Form einer rechteckigen Matte vorliegt, auf der die Person liegen kann, und dass sie eine Antennenanordnung umfasst, die mindestens fünf erste Antennen und mindestens fünf zweite Antennen umfasst, die alle in Reihe versorgt werden und pflasterartig angeordnet sind.

11. Vorrichtung nach Anspruch 1, **dadurch gekennzeichnet, dass** sie in Form eines Riemens vorliegt, der dazu bestimmt ist, um die Hände getragen zu werden, wobei der Handriemen einen rechteckigen Träger und eine Antennenanordnung umfasst, die drei erste Antennen und drei zweite Antennen umfasst, die gemäß dem Umfang des Trägers angeordnet sind, wobei sich die erste Antenne mit der zweiten Antenne in der Anordnung abwechselt.

12. Vorrichtung nach Anspruch 1, **dadurch gekennzeichnet, dass** sie in Form eines Armbands vorliegt, das dazu bestimmt ist, um den Arm oder den Knöchel getragen zu werden, wobei das Armband einen rechteckigen Träger, die erste Antenne und die zweite Antenne umfasst.

## Claims

1. A device (100 ; 200; 300 ; 400 ; 500; 600) for emitting a magnetic field comprising:
- an insulating support (102 ; 202 ; 302 ; 402 ; 502; 602) intended to be put into contact with a part of the person's body,
- at least one first antenna (104a; 204a) and at least one second antenna (104b; 204 b), each antenna being a coil of conductive metal wire wound along the outline of a hexagon, the length of its side being between 2 cm and 20 cm,
- a power source (106; 206; 306; 406; 506; 606) with antenna current,
the first antenna, the second antenna and the power source being integral with the insulating support,
the current being alternating and the first antenna and the second antenna being wound in opposite directions.

2. The device according to Claim 1, **characterized in that** the hexagon is regular, and that the length of its side is between 3 cm and 15 cm.

3. The device according to Claim 1, **characterized in that** it comprises at least three antennas arranged in a paving.

4. The device according to Claim 1, **characterized in that** the first antenna and the second antenna are aligned along their major axis.

5. The device according to Claim 1, **characterized in that** the first antenna and the second antenna are aligned along their shortest axis.

6. The device according to Claim 1, **characterized in that** the first antenna and the second antenna are directly electrically connected.

7. The device according to claim 1, **characterized in that** the part of the person's body is chosen in the group consisting of head, face, neck, arm, back, waist, abdomen, lumbar region, hip, thigh, calf and ankle.

8. The device according to claim 1, in the form of a belt or trousers, **characterized in that** it comprises at least two sets of antennas connected in parallel, **in that** each set comprising at least two antennas connected in series and **in that** each set comprises at least the first antenna and the second antenna.

9. The device according to claim 1, in the form of a face strap comprising a set of antennas connected in series comprising at least the first antenna and the second antenna, the antennas of the set being distributed in groups of three , and the antennae of a group being arranged in a paving.

10. The device according to claim 1, **characterized in that** it is in the form of a rectangular mat on which the person can lie down, and that it comprises a set of antennas comprising at least five first antennas and at least five second antennas, all fed in series and arranged in a paving.

11. The device according to claim 1, **characterized in that** it is in the form of a strap intended to be worn around the hands, said hand strap comprising a rectangular support and a set of antennae comprising three first antennas and three second antennas arranged along the perimeter of the support, the first antenna alternating with the second antenna in the assembly.

12. The device according to claim 1, **characterized in that** it is in the form of a bracelet intended to be worn around the arm or the ankle, said bracelet comprising a rectangular support, the first antenna and the second antenna.
